# EUROPEAN PATENT APPLICATION

(11) **EP 4 772 201 A1**
(43) Date of publication of application: **08.07.2026**
(21) Application number: 25225669.8
(22) Date of filing: 19.12.2025
(51) Int. Cl.: A61L 27/06, A61L 27/42, A61L 27/50, B33Y 70/10

(54) **AM OPTIMIZED DISPERSION STRENGTH BETA-TITANIUM ALLOY**

(30) Priority: 07.01.2025 US 202563742505 P
(71) Applicant: Howmedica Osteonics Corp., Mahwah, NJ 07430 (US)
(72) Inventor: HEARD, David William, SUMMIT, 07901 (US); HANSON, Keenan Michael, SLOATSBURG, 10974 (US)
(74) Representative: Regimbeau

(57) **Abstract**

Disclosed herein is a biocompatible composite material and a method of preparing a powder containing same. The biocompatible material can include a matrix and a second-phase dispersion. The matrix can include primary particles forming a titanium alloy. The second-phase dispersion can include secondary particles each encompassed by one of the primary particles of the matrix. The method can include preparing an ingot having the biocompatible material and atomizing the ingot to produce a composite material powder.

## Description

### CROSS REFERENCE TO RELATED APPLICATION

This application claims the benefit of the filing date of United States Provisional Application No. 63/742,505, filed on January 7, 2025, the disclosure of which is hereby incorporated by reference.

### FIELD OF THE INVENTION

The present disclosure relates to a biocompatible composite material and a method of preparing a powder containing the same.

### BACKGROUND OF THE INVENTION

A total knee revision (TKR) procedure is a commonly performed surgery that is on track to increase in the future. However, many complications can arise during TKR procedures due to significant bone loss from excessive bone resection. Excessive bone resection during TKR procedures may result in the need to use allografts, cone augments, or metaphyseal sleeves to stabilize the bone. These remedies greatly increase the costs and complexity of TKR procedures. As such, tools and procedures that minimize the amount of resected bone during a total knee arthroplasty (TKA) procedure and thereby preserve as much bone stock as possible for a later revision are in high demand.

One current approach to minimize resected bone during a TKA procedure is to reduce the thickness of either a femoral or a tibial component of an implant. Using thinner components results in significant bone sparing and thereby increased bone stock to be utilized by a surgeon in case of a future revision. However, these components, typically fabricated using a Ti-6Al-4V alloy, are fatigue-limited. To compensate for reductions in fatigue strength caused by reducing component thickness, higher fatigue strength material have been utilized in fabricating these components. One such material, a β-titanium alloy, Ti-12Mo-6Zr-2Fe (TMZF), was developed due to its improved biocompatibility and greater fatigue life over Ti-6Al-4V, with the TMZF having an approximately 45% increase in notched fatigue life over that of Ti-6Al-4V.

However, according to Al-Bermani, S.S., et al. "The origin of microstructural diversity, texture, and mechanical properties in electron beam melted Ti-6Al-4V," Metallurgical and materials transactions A, 41.13 (2010): 3422-3434, the disclosure of which is hereby incorporated herein by reference in its entirety, use of Ti-6Al-4V alloy powder for additive manufacturing (AM) at elevated temperatures results in coarse alpha laths and high levels of metallographic texture, which is associated with a reduction in fatigue strength. Additionally, a study conducted by Mantri and Banerjee has shown that β-titanium alloys form large columnar grains during Laser Based Direct Metal Deposition (L-DMD) processing. *See* Mantri, S. A., and R. Banerjee. "Microstructure and micro-texture evolution of additively manufactured β-Ti alloys." Additive Manufacturing 23 (2018): 86-98, the disclosure of which is incorporated herein in its entirety.

### BRIEF SUMMARY OF THE INVENTION

In accordance with an aspect of the present disclosure, a biocompatible composite material is provided. The biocompatible material according to this aspect may include a matrix having primary particles forming a titanium alloy and a second-phase dispersion including secondary particles each encompassed by one of the primary particles of the matrix.

Continuing in accordance with this aspect, the primary particles may form a metastable β-titanium alloy. The metastable β-titanium alloy may include Ti-12Mo-6Zr-2Fe (TMZF) or Ti-35Nb-7Zr-5Ta (TNZT). The primary particles may include titanium particles and beta stabilizing particles separate from the titanium particles. The secondary particles may be encompassed by the titanium particles. The secondary particles may be encompassed by the beta stabilizing particles. The beta stabilizing particles may be made of elements including at least one of or a combination of Mo, Zr, Fe, Nb, or Ta.

Continuing in accordance with this aspect, the matrix may include a homogeneous distribution of one or more of the secondary particles within the composite material. The primary particles of the matrix may be homogenously distributed within the composite material. The composite material may be a powder.

Continuing in accordance with this aspect, the second-phase dispersion may be in the form of an oxide, a ceramic, a carbide, or a boride particle. The secondary particles may be made of molecules having a chemical formula of TiB₂, TiC, or SiC.

Continuing in accordance with this aspect, the secondary particles may be made of molecules having a chemical formula of ZrO₂, TiO₂, AlO₂, Y₂O₃, SiO₂, and Er₂O₃.

In accordance with another aspect of the present disclosure, a composite material is provided. A composite material according to this aspect may include a matrix having primary particles forming metastable Ti-12Mo-6Zr-2Fe (TMZF) in the β-phase and a second-phase dispersion including a homogeneous distribution of secondary oxide particles each encompassed by one of the primary particles of the matrix.

Continuing in accordance with this aspect, the second-phase dispersion may be configured to form a refined grain-structure comprising β-titanium and a dispersion phase.

In accordance with another aspect of the present disclosure, a method of preparing a composite material powder is provided. A method according to this aspect may include the steps of preparing an ingot including a composite material and atomizing the ingot to produce a composite material powder. The composite material may include a matrix having primary particles forming a metal alloy and a second-phase dispersion including secondary particles each encompassed by one of the primary particles of the matrix.

Continuing in accordance with this aspect, the step of preparing the ingot may include Vacuum Induction Melting, Vacuum Arc (Re-)Melting, or Induction Skill Melting of the composite material.

Continuing in accordance with this aspect, the step of preparing the ingot may include mechanical alloying, metal injection molding, or compaction and sintering of the composite material.

Continuing in accordance with this aspect, the composite material powder may include a composite material particle that is fully pre-alloyed.

Continuing in accordance with this aspect, the method of preparing the composite material powder may include a step of sieving the composite material powder to isolate powder particles of the composite material powder having a diameter in a range of 10-50 µm.

Continuing in accordance with this aspect, the method of preparing the composite material powder may include a step of sieving the composite material powder to isolate powder particles of the composite material powder having a diameter in a range of 45-150 µm.

Continuing in accordance with this aspect, the method of preparing the composite material powder may include a step of forming the composite material powder into a component via a Laser Power Bed Fusion process or an Electron Beam Powder Bed Fusion process. The component is a femoral component or a tibial component of an implant.

Continuing in accordance with this aspect, the primary particles of the matrix may form a titanium alloy. The step of forming the composite material into the component may refine a grain structure formed during the solidification of the composite material and may be defined by a β-phase titanium alloy and the second-phase dispersion.

Continuing in accordance with this aspect, the method of preparing the composite material powder may include a step of heat treating an in-process component structure before fully forming the component to cause the precipitation of a third *α*-phase of the matrix. The heat-treating step may include controlling the size and location of the metal alloy using an isothermal, a high-low, or a low-high thermal profile. The heat-treating step may transform a microstructure of the in-process component structure from a grain structure of entirely β-phase grains defined by the metal alloy with secondary particles into a grain structure with fine *α*-phase precipitates of the metal alloy throughout the β-phase grains.

Continuing in accordance with this aspect, the primary particles may form a metastable β-titanium alloy including beta stabilizing particles. The metastable β-titanium alloy may include Ti-12Mo-6Zr-2Fe (TMZF) or Ti-35Nb-7Zr-5Ta (TNZT). The beta stabilizing particles may be made of elements including at least one of or a combination of Mo, Zr, Fe, Nb, or Ta.

Continuing in accordance with this aspect, the matrix may include a homogenous distribution of one or more of the secondary particles within the composite material. The primary particles of the matrix may be homogeneously distributed within the composite material. The composite material may be biocompatible. The second-phase dispersion may be in the form of an oxide, a ceramic, a carbide, or a boride particle.

Continuing in accordance with this aspect, the secondary particles may be made of molecules having a chemical formula of TiB₂, TiC, or SiC.

Continuing in accordance with this aspect, the secondary particles may be made of molecules having a chemical formula of ZrO₂, TiO₂, AlO₂, Y₂O₃, SiO₂, and Er₂O₃.

### BRIEF DESCRIPTION OF THE DRAWINGS

A more complete appreciation of the subject matter of the present invention and of the various advantages thereof can be realized by reference to the following detailed description in which reference is made to the accompanying drawings in which:
FIG. 1 is a diagram illustrating a microstructure of a composite material powder of the present disclosure.
FIG. 2 is a chart illustrating a method of preparing the composite material powder of FIG. 1 and building a component using the composite material powder.
FIG. 3 is a diagram illustrating a grain structure development of the composite material powder of FIG. 1 after additive manufacturing.
FIG. 4 is a diagram illustrating a grain structure development in a composite material in correlation with a thermal aging time-temperature profile during an isothermal heat treatment during the step of building the component of FIG. 2.
FIG. 5A is a diagram illustrating a grain structure development in a composite material in correlation with a thermal aging time-temperature profile during a two-step, high-low heat treatment during the step of building the component of FIG. 2.
FIG. 5B is a diagram illustrating a grain structure development in a composite material in correlation with a thermal aging time-temperature profile during a two-step, low-high heat treatment during the step of building the component of FIG. 2.

### DETAILED DESCRIPTION

As used herein, the terms "about," "generally," and "substantially" are intended to mean that slight deviations from absolute are included within the scope of the term so modified. To aid the Patent Office and any readers of any patent issued on this application in interpreting the claims appended hereto, Applicant notes that it does not intend any of the appended claims or claim elements to invoke 35 U.S.C. § 112(f) unless the words "means for" or "step for" are explicitly used in the particular claim.

In describing the preferred aspects of the disclosure, specific terminology will be used for the sake of clarity. However, the disclosure is not intended to be limited to any specific terms used herein, and it is to be understood that each specific term includes all technical equivalents, which operate in a similar manner to accomplish a similar purpose.

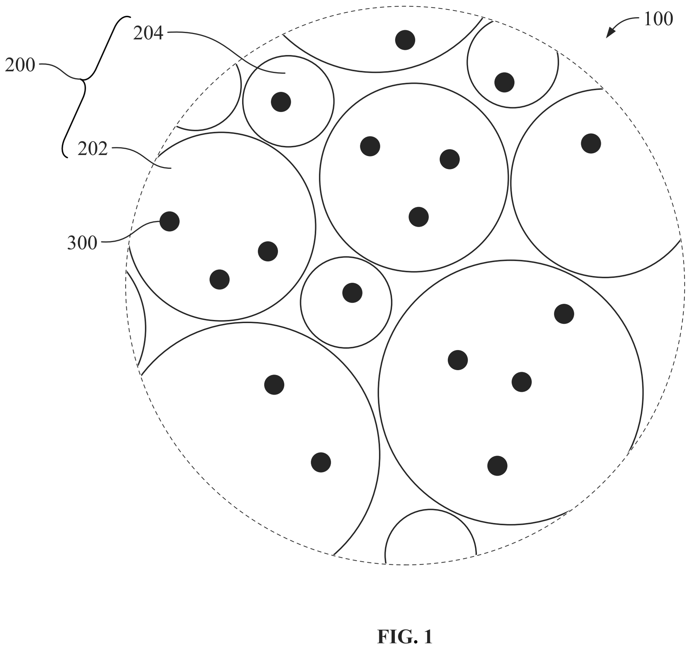

Referring to FIG. 1, a biocompatible composite material, such as a biocompatible composite material powder 100, comprises a matrix 200 including primary particles 202 and 204. A second phase dispersion including secondary particles 300 is encompassed by one of primary particles 202 and 204 of matrix 200. Biocompatible composite material is a metal matrix composite, wherein matrix 200 includes primary particles 202 and 204 that form a titanium alloy. The second phase dispersion is integrated within matrix 200. Primary particles 202 and 204 form a metastable β-titanium alloy including titanium particles 202 and β-stabilizing particles 204. Metastable β-titanium alloy includes Ti-12Mo-6Zr-2Fe (TMZF), Ti-35Nb-7Zr-5Ta (TNZT), or similar biocompatible materials. β-stabilizing particles 204 include elements that can include, singularly or in combination, Mo, Zr, Fe, Nb, or Ta.

As best shown in FIG. 1, biocompatible composite material powder 100 includes a homogenous distribution of one or more secondary particles 300 encompassed within matrix 200. Matrix 200 also includes homogenous distribution of primary particles 202 and 204 within composite material. Secondary particles 300 are encompassed by both titanium particles 202 and β-stabilizing particles 204 that make up primary particles 202 and 204 or metastable β-titanium alloy. Homogenous distribution includes a uniform dispersion of secondary particles 300 throughout the matrix.

Biocompatible composite material powder 100 includes second-phase dispersion in the form of an oxide, ceramic, carbide, or boride particle. In one aspect, composite material may have a composition having a formula of Ti-12Mo-6Zr-2Fe-XM, wherein X ranges from 0.5 to 1.0 and M represents secondary particles 300. In one aspect, secondary particles 300 can be an oxide such as ZrO2, TiO2, AlO2, Y2O3, SiO2, Er2O3, or any other oxide phase. In another aspect, secondary particles can be a ceramic, carbide, or boride phase such as TiB2, TiC, SiC, etc. The biocompatible composite material can be a composite material powder, with each powder particle having a desired size ranging from 10 to 150 µm. Each powder particle can be fully pre-alloyed, ensuring that it contains all elements of the composite material.

In another aspect of the composite material powder 100 of the present disclosure, composite material can include matrix 200 having primary particles 202 and 204 that form metastable Ti-12Mo-6Zr-2Fe (TMZF) in β-phase. This matrix 200 is enhanced by second-phase dispersion including a homogeneous distribution of secondary oxide particles 300, each encompassed by one of primary particles 202 and 404 of matrix. In some aspects, the second-phase dispersion is specifically configured to form a refined grain-structure comprising of β-titanium and a dispersion phase particle.

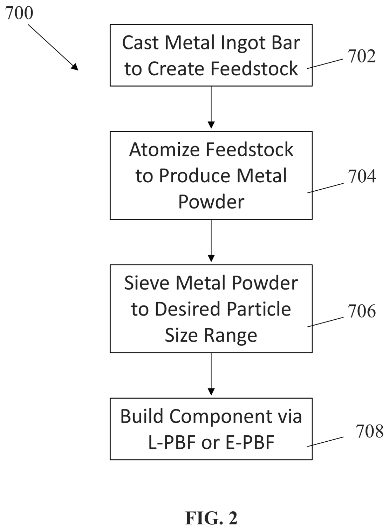

A method 700 of preparing a composite material powder according to an aspect of the present disclosure is shown in FIGS. 2. This method can include the steps of preparing an ingot containing the composite material and atomizing the ingot to produce the composite material powder. The composite material powder 100 can include matrix 200 having primary particles 202 and 204 forming metal alloy and second-phase dispersion including secondary particles 300 each encompassed by one of primary particles of matrix.

As shown in FIG. 2, method 700 includes a step 702 of casting a metal ingot bar to create feedstock, a step 704 of atomizing the feedstock to produce a metal powder, a step 706 of sieving metal powder to a desired particle size range, and a step 708 of building a component via a Laser Power Bed Fusion, Electron Beam Powder Bed Fusion, or any other similar process 708.

Step 702 of casting metal ingot bar to create feedstock can be performed by suitable metallurgical techniques. These can range from skull melting - a process that leverages a water-cooled metal mold to contain the molten metal without it coming into contact with the crucible - to plasma arc remelting, which involves the use of a plasma torch for precision melting. Additional techniques can include electron beam melting, utilizing focused beams of electrons for high-purity melting processes under vacuum conditions, as well as Vacuum Induction Melting (VIM) where an electric current creates an inductive magnetic field to heat and melt the metal in a vacuum chamber. Vacuum Arc Remelting (VAR) or Induction Skull Melting (ISM) can also be employed in step 702 to refine the composite material's microstructure and improve its homogeneity. In alternative aspects, the preparation of the ingot can include mechanical alloying, which is a solid-state powder processing technique involving repeated cold welding, fracturing, and rewelding of powder particles, or metal injection molding, where metal powders are mixed with a binder to create a feedstock that is injection molded, debindered, and finally sintered. Another variant includes the compaction and sintering of composite materials to achieve the desired shape and mechanical properties. In another aspect, the ingot can be prepare using powder metallurgy techniques, wherein primary and secondary particles are compressed to create a compact that is then encased or enveloped in titanium foil to form the ingot. Alternatively, the mixture of primary and secondary particles can bypass traditional ingot formation and instead be directly exposed to a plasma spheroidization process. Here, they are rapidly melted and cooled to form spherical particles that are particularly suited for powder bed fusion processes.

For the ingot created in step 702, the specific composition includes a blend of primary and secondary particles, with the secondary particles making up a minor yet crucial fraction ranging from 0.1 to 2 weight % of the total mixture. This carefully regulated proportion ensures the desired properties and behavior of the final composite material.

Step 704 includes feeding ingot of step 702 into an atomizer to produce the composite material powder. This process can include the injection of the ingot into the core of the atomizer, where it is then converted into a fine composite material powder. During this procedure, secondary particles are effectively transported and dispersed within a high-velocity stream of the molten primary particles as they are ejected from the nozzle of the atomizer. The atomization process can be controlled and executed at a specific thermal condition. For example, the temperature can be maintained above the melting point of the primary particles while keeping it below the melting point of the secondary particles. This disparity in temperature thresholds allow the secondary particles to remain solid and retain their integrity while the primary particles assume a liquid state. This ensures that, upon leaving the nozzle, the primary and secondary particles solidify rapidly, effectively freezing the secondary particles within the matrix of the primary particles as they cool. As a result, each secondary particle is encapsulated within the primary particles at the time of atomization, thus creating composite material particles where the secondary particles are locked into place.

It should be understood that every particle within the resultant composite material powder is fully pre-alloyed - *i.e.,* they contain all the constituent elements of the composite material. This process ensures a consistent and homogeneous distribution of the secondary particles throughout the entirety of the composite material powder. Although not every primary particle will contain a secondary particle within it after the atomization process, those primary particles that do incorporate secondary particles will contribute to a homogeneous distribution throughout the composite material powder.

After ingot formation and atomization of composite material powder, in step 706 composite material powder is sieved to desired particle size range. In some aspects of the present disclosure, composite material powder is sieved to isolate powder particles of composite material powder having diameter ranging from 10 to 50 µm. In some aspects, composite material powder is sieved to isolate powder particles of composite material powder having diameter ranging from 15 to 50 µm. In other aspects of the present disclosure, composite material powder is sieved to isolate powder particles of composite material powder having diameter ranging from 45 to 150 µm.

A step 708 of building component includes a Laser Power Bed Fusion or an Electron Beam Powder Bed Fusion process 708. Forming component from composite material powder further includes Laser Power Bed Fusion process or Electron Beam Powder Bed Fusion process. Desired particle size range is 10 to 50 µm for Laser Power Bed Fusion process and 45 to 150 µm for Electron Beam Powder Bed Fusion. Resulting component may be a femoral component or a tibial component of an implant with refined grain structure and increased fatigue strength. Additional implant components may also be manufactured using process of present disclosure.

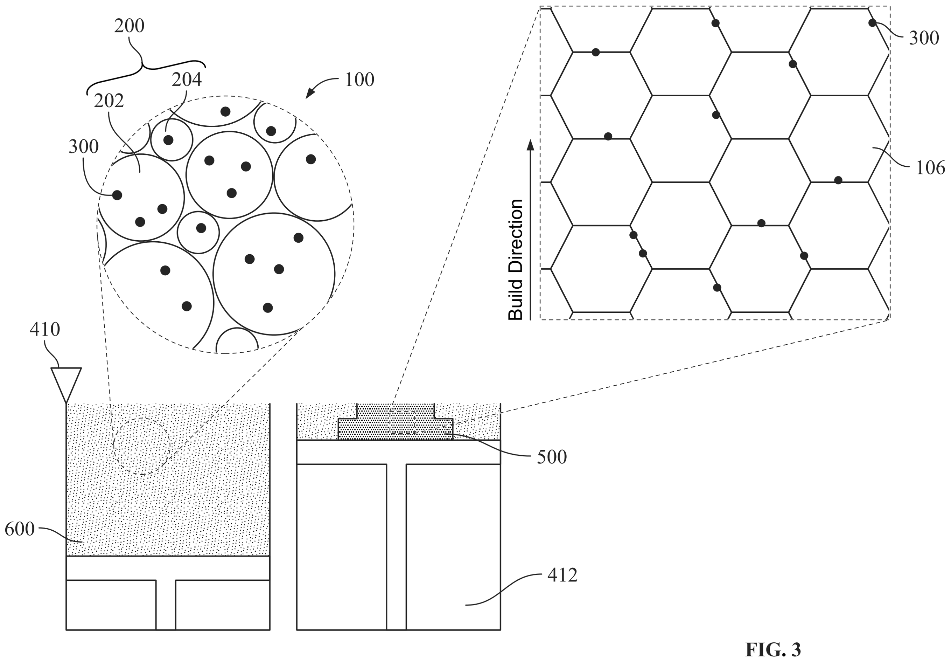

As shown in FIG. 3, component 500 contains equiaxed β-phase grains 106 developed during additive manufacturing of metastable β-Ti alloy powder. Primary particles 202 and 204 of matrix 200 form titanium alloy. Forming composite material powder 100 into component 500 not only refines the grain structure that emerges during the solidification of the composite material but also solidifies the definition brought about by the β-phase titanium alloy coupled with the second phase dispersion. Laser Power Bed Fusion (LPBF), Electron Beam Powder Bed Fusion (EBPBF) or other similar metal additive manufacturing techniques can be used manufacture complex metal components 500. These technologies enable the precision crafting of elaborate geometries that would be challenging to achieve through conventional manufacturing methods. Composite material when solidified via an additive manufacturing flow such as Laser Power Bed Fusion or Electron Beam Powder Bed Fusion will form refined grain structure consisting of β-titanium and dispersion phase with equiaxed β-phase grains 106 while avoiding formation of large columnar grains. During the additive manufacturing of component 500, a re-coater blade 410 is used to apply pressure to composite material powder in a powder reservoir 600 above a build chamber 412. Re-coater blade 410 is systematically operated to apply consistent, uniform pressure to the powdered composite material stored in powder reservoir 600. This evenly distributes the powder over build chamber 412, preparing each new layer for selective melting and subsequent solidification into component 500. This continuous and precise re-coating provides the desired tight tolerances and surface finish of the final component.

As best shown in FIG. 3, after the additive manufacturing process concludes and component 500 emerges, the distribution of secondary particles 300 is no longer homogeneous within the matrix. This is due to the dynamic interactions and conditions during the melting and subsequent solidification phases. The homogenous dispersal of secondary particles before the manufacturing process now translates into a more varied distribution within the resolidified matrix, resulting in each manufactured component 500 possessing its unique microstructural characteristics revealing a more random localization of the secondary particles in the newly formed matrix of the component.

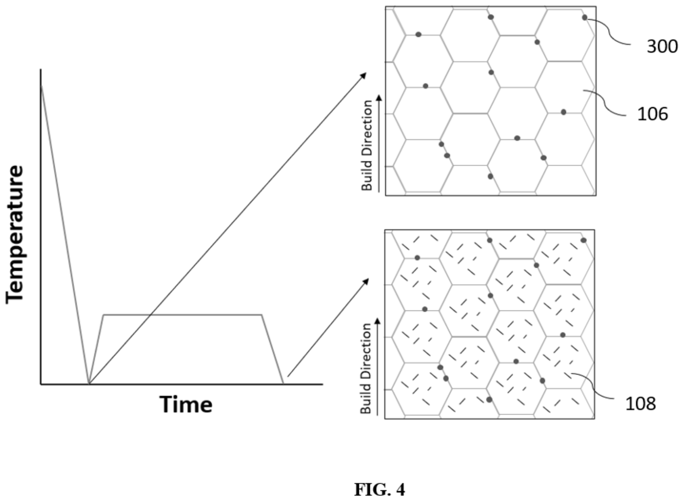

The composite material can be subjected to a specific thermal aging regimen or heat treatment method that follows a precise time-temperature trajectory. As shown in FIG. 4, the thermal aging regimen may include an isothermal profile. In the isothermal profile, β-annealing is performed at a temperature above a β-transus temperature. Upon completion of the β-annealing, the composite material is cooled to room temperature prior to being subjected to an aging treatment. The aging treatment includes a secondary thermal treatment at a temperature within the α+β region of the composite material. Such a process results in the precipitation of a third α-phase within a β-matrix.

This intentional heat treatment is designed to modify the original microstructure of the composite material, which initially consists solely of a β-matrix having equiaxed β-phase grains 106 populated with secondary particles 300. Through this thermal process during the component-building step, the material undergoes a transformation that results in the emergence of fine α-precipitates 108, that are evenly distributed throughout the equiaxed β-phase grains 106. Initially characterized by a grain structure composed entirely of β-phase grains 106, defined by the metal alloy with secondary particles 300, this structure is transformed to include fine α-phase precipitates 108 dispersed throughout the β-phase grains. Such fine α-phase precipitates provide a higher degree of strengthening due to an increased surface area to volume ratio.

The time and temperature of the secondary thermal treatment can be selected based on the desired size, distribution, and morphology of the α-phase desired. The precipitation of the third α-phase further strengthens the composite material. These treatments provide specific microstructural changes within the material, notably the induction of the third α-phase. The emergence of this phase enhances the overall strength of the composite material. By applying heat treatment protocols to the intermediate structure of the component, prior to its full formation into component 500, the material undergoes a transformative process that leads to the precipitation of this third α-phase within the matrix. The precipitated third α-phase represents a fundamental alteration that fortifies the material, optimizing the final component for high-performance applications that require robust mechanical properties.

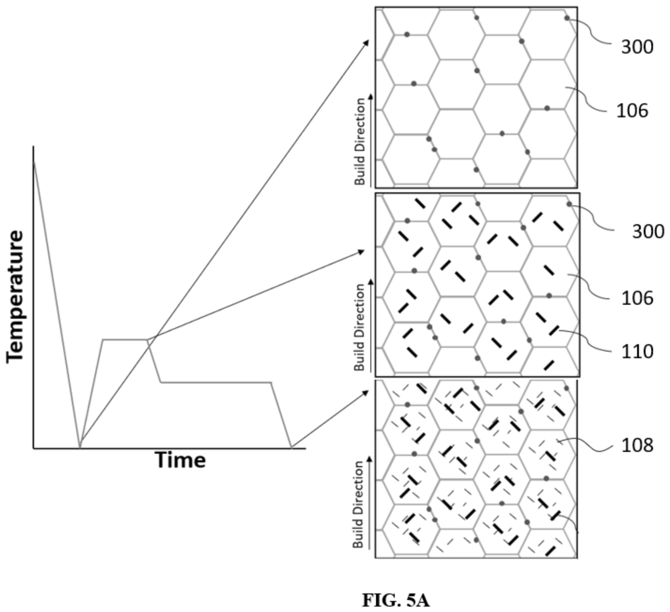

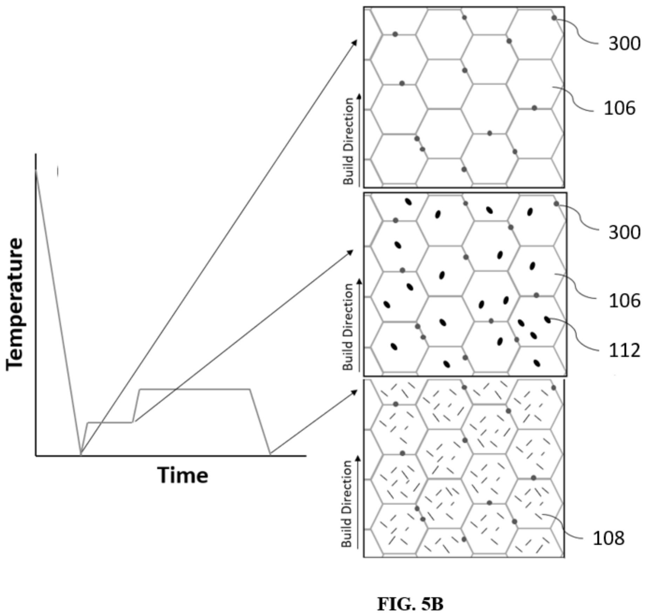

FIGS. 5A-5B show other examples of thermal aging regimens to control size and placement of the particles of the metal alloy. Various thermal profiles, such as a high-low thermal gradient (FIG. 5A) or a low-high thermal gradient (FIG. 5B), can also be used to alter the microstructure of component 500 and to precipitate the third α-phase.

As shown in FIG. 5A, the thermal aging regimen may include a high-low thermal profile. In the high-low thermal profile, β-annealing is performed at a temperature above a β-transus temperature. Upon completion of the β-annealing, the composite material is cooled to room temperature prior to being subjected to a secondary thermal treatment. The secondary thermal treatment may include a first and a second aging treatment. The composite material is subjected to the first aging treatment at a temperature within the α+β region of the alloy. The composite material is held at this temperature for a period of time prior to being cooled to a lower aging temperature within α+β region of the alloy for a second aging treatment. The first aging treatment results in the formation of a coarse α-phase within the β-matrix, while the second aging treatment results in the precipitation of fine α-phase precipitates 108 within the β-matrix. The time and temperature of both the first and second aging treatments can be selected based on the desired size, distribution, and morphology of α-phase desired. Initially characterized by a grain structure composed entirely of β-phase grains 106, defined by the metal alloy with secondary particles 300, this structure is transformed to include coarse α-phase precipitates 110 dispersed throughout the β-phase grains during the first aging treatment. During the second aging treatment, this structure is transformed to also include fine α-phase precipitates 108.

As shown in FIG. 5B, the thermal aging regimen may include a low-high thermal profile. In the low-high thermal profile, β-annealing is performed at a temperature above a β-transus temperature. Upon completion of the β-annealing, the alloy is cooled to room temperature prior to being subjected to a secondary thermal treatment. The secondary thermal treatment may include a first and a second aging treatment. The composite material is subjected to the first aging treatment at a temperature within the α+β region of the alloy. The composite material is held at this temperature for a period of time prior to being heated to a higher aging temperature within α+β region of the alloy for a second aging treatment. The first aging treatment results in the formation of precursor phases to the α-phase, such as ω-phase and β'-phase within the β-matrix. The second aging treatment aging results in the transformation of the precursor phase precipitates 112 to fine α-phase precipitates 108 within the β-matrix with the increase homogeneity with respect to the isothermal profile. The time and temperature of both the first and second aging treatments can be selected based on the desired size, distribution, and morphology of α-phase desired. Initially characterized by a grain structure composed entirely of β-phase grains 106, defined by the metal alloy with secondary particles 300, this structure is transformed to include precursor phase precipitates 112 dispersed throughout the β-phase grains during the first aging treatment. During the second aging treatment, this structure is transformed to include fine α-phase precipitates 108, absent the precursor phase precipitates 112.

The mechanical performance of component 500 can be finely tuned by adjusting secondary thermal treatment parameters such as the duration and temperature of the heat treatment. This strategic modification allows for enhanced control over the material properties and performance of the final product.

Although the invention herein has been described with reference to particular examples, it is to be understood that these examples are merely illustrative of the principles and applications of the present invention. For example, features described in relation to one particular aspect may be combined with features of other aspects described herein. It is therefore to be understood that numerous modifications may be made to the illustrative examples and that other arrangements may be devised without departing from the spirit and scope of the present invention as defined in the appended claims.

## Claims

1. A biocompatible composite material comprising:
a matrix including primary particles forming a titanium alloy; and
a second-phase dispersion including secondary particles each encompassed by one of the primary particles of the matrix.

2. The biocompatible composite material according to claim 1, wherein the primary particles form a metastable β-titanium alloy.

3. The biocompatible composite material according to claim 2, wherein the metastable β-titanium alloy includes Ti-12Mo-6Zr-2Fe (TMZF) or Ti-35Nb-7Zr-5Ta (TNZT).

4. The biocompatible composite material according to any one of claims 1-3, wherein the primary particles include titanium particles and beta stabilizing particles separate from the titanium particles.

5. The biocompatible composite material according to claim 4, wherein the secondary particles are encompassed by the titanium particles.

6. The biocompatible composite material according to claim 4 or claim 5, wherein the secondary particles are encompassed by the beta stabilizing particles.

7. The biocompatible composite material according to any one of claims 4-6, wherein the beta stabilizing particles are made of elements including at least one of or a combination of Mo, Zr, Fe, Nb, or Ta.

8. The biocompatible composite material according to any one of claims 1-7, wherein the matrix includes a homogeneous distribution of one or more of the secondary particles within the composite material.

9. The biocompatible composite material according to any one of claims 1-8, wherein the primary particles of the matrix are homogenously distributed within the composite material.

10. The biocompatible composite material according to any one of claims 1-9, wherein the composite material is a powder.

11. The biocompatible composite material according to any one of claims 1-10, wherein the second-phase dispersion is in the form of an oxide, a ceramic, a carbide, or a boride particle.

12. The biocompatible composite material according to any one of claims 1-11, wherein the secondary particles are made of molecules having a chemical formula of TiB₂, TiC, or SiC.

13. The biocompatible composite material according to any one of claims 1-11, wherein the secondary particles are made of molecules having a chemical formula of ZrO₂, TiO₂, AlO₂, Y₂O₃, SiO₂, and Er₂O₃.

14. A method of preparing a composite material powder, comprising steps of:
preparing an ingot comprising a composite material; and
atomizing the ingot to produce a composite material powder,
wherein the composite material comprises:
a matrix including primary particles forming a metal alloy; and
a second-phase dispersion including secondary particles each encompassed by one of the primary particles of the matrix.

15. The method according to claim 14, wherein the composite material powder
includes a composite material particle that is fully pre-alloyed.
